# EUROPEAN PATENT SPECIFICATION

(11) **EP 1 559 703 B1**
(45) Date of publication and mention of the grant of the patent: **17.12.2008**
(21) Application number: 02774265.9
(22) Date of filing: 17.10.2002
(51) Int. Cl.: C07C 43/196, C07C 41/34, A61P 3/10

(54) **A NATURAL COMPOUND USEFUL FOR TREATING DIABETES, ITS PREPARATION AND USE**
NATÜRLICHE VERBINDUNG FÜR DIE BEHANDLUNG VON DIABETES, DEREN HERSTELLUNG UND VERWENDUNG
COMPOSE NATUREL SERVANT DANS LE TRAITEMENT DE DIABETES, SA PREPARATION ET SON UTILISATION

(43) Date of publication of application: 03.08.2005
(73) Proprietor: Liang, Jingyu, Jiangsu 210009 (CN); Sun, Mingjie, Chenzhen City, Guangdong 518000 (CN)
(72) Inventor: LIANG, Jingyu, Nanjing, Jiangsu 210009 (CN); WU, Feihua, Dept. of Pharmacology, Nanjing, Jiangsu 210009 (CN); BAO, Guanhu, Mail Box 224, Nanjing, Jiangsu 210009 (CN); CHENG, Qilei, Dept. of Pharmacology, Nanjing, Jiangsu 210009 (CN)
(74) Representative: Olsson, Stefan
(86) International application number: PCT/CN2002/000728
(87) International publication number: WO 2004/039759

(56) References cited:
- DATABASE CA [Online] CHEMICAL ABSTRACTS SERVICE, COLUMBUS, OHIO, US; LEE, CHUEN LAI ET AL: "Inhibitory effect of Taxus mairei heartwood extractives on the curing of unsaturated polyester resins" XP002373313 retrieved from STN Database accession no. 1975:533681 & MOKUZAI GAKKAISHI , 21(4), 249-56 CODEN: MKZGA7; ISSN: 0021-4795, 1975,
- PLOUVIER, VICTOR: "Sur la recherche du séquoyitol et du pinitol chez quelques Gymnospermes" COMPTES RENDUS, vol. 245, 1957, pages 2377-2379, XP008061922
- MURAKAMI ET AL.: 'Chemical and chemotaxonomical studies on filices. LVI' YAKUGAKU ZASSHI vol. 105, no. 7, 1995, pages 649 - 654, XP002987534
- SULTANA N. ET AL.: 'Two novel prenylated flavones from the aerial parts of melicope micrococca' PHYTOCHEMISTRY vol. 50, no. 7, 10 April 1999, pages 1249 - 1253, XP004290550
- DITTRICH P. ET AL.: 'D-1-O-methyl-mucoinositol in higher plants' PHYTOCHEMISTRY vol. 11, no. 1, January 1971, pages 245 - 250, XP002999251
- ANDERSON LAURENS ET AL.: 'Inositol methyl ethers, II, the formula of sequoyitol' J. AM. CHEM. SOC. vol. 79, no. 5, 05 March 1957, pages 1171 - 1174, XP002987535
- NARAYANAN C.R. ET AL: 'PINITOL - A NEW ANTI-DIABETIC COMPOUND FROM THE LEAVES OF BOUGAINVILLEA SPECTABI LIS' CURRENT SCIENCE vol. 56, no. 3, 1987, pages 139 - 141, XP000577900

## Description

### Technical field

The present invention relates to a natural compound, 5-*O*-methyl-*myo*-inositol (Sequoyitol), which is extracted and separated from *Taxus spp*, in particular *Taxus yunnanensis* Cheng et L. K. Fu, *Taxus chinensis* var. *mairei* (Lemee et Levl) Cheng et L. K. Fu, etc., to a process for preparing the same, and to pharmaceutical use thereof.

### Background art

With the acceleration of aging proceeding of population in the world, diabetes is a common and frequently occurring disease. At present, the pathogenesis of diabetes is still not clear, and the treatment for diabetes is mainly to alleviate hyperglycemia and to prevent complications by using drugs. The inventions and applications of insulin and main chemically synthetic hypoglycemic agents for oral administration are helpful to diabetics, but they per se still have serious side effects such as hypoglycemia, lactic acid intoxication, etc., which greatly limit their uses and therapeutic effects (Wang Zhongxiao, Journal of Shanxi Medical University, 31 (6), 2000, pages 555-556). Thus, it is urgent to search and develop novel hypoglycemic substances with high performance, low toxicity, and high safety and reliability.

According to Chinese medical documents, Taxus leaves have functions of diuresis, dredge the meridian passage, and can be used for treatment of diabetes (Glossary of Herbs and Drugs in China, the last volume (2nd edition), Edited by Xie Zongwan, People' Medical Publishing House, page 722), and Taxus has special effect for treatment of diabetes (Gan Weisong, pharmaceutical botany, 1991, page 140), but the modem studies on antidiabetic components of Taxus and activity thereof are not found in the prior art. Pinitol has been proposed for the use as an anti-diabetic compound (Navayanan et al., Current Science, vol. 56, no. 3, 1987, pages 139-141, XP000577900). The occurrence of sequoyitol in Taxus species is known from e.g. Plouvier, Victor: "Sur la recherche du sequoyitol et du pinitol chez quelquez Gymnospermes" Comptes Rendus, vol. 245, 1957, pages 2377-2379, XP008061922. We deeply studied the antidiabetic active components of Taxus and find that the natural compound, 5-*O*-methyl-*myo*-inositol (Sequoyitol), is an antidiabetic active component having notable activity and very low toxicity, and has the following formula(I):

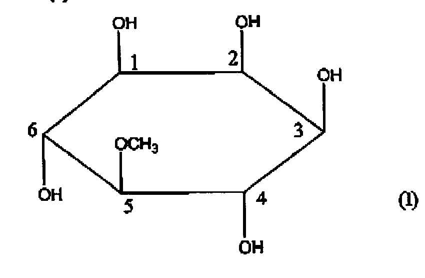

The mother nucleus of Sequoyitol (5-*O*-methyl-*myo*-inositol) is *myo*-inositol, which is one of stereoisomers of cyclohexanol (cyclohexanol has several chiral centers, and thus has several stereoisomers). Some articles state that Sequoyitol exists in plants such as *Taxus*, *Pinus*, *Cypress*, *Cephalotaxus fortunei*, *Taxodiaceae*, etc. (Phytochemistry, 1971, 11:245-250). The structure of pentaacetyl derivative of Sequoyitol was identified by high resolution ¹H-NMR (Phytochemistry, 27(1):279-181, 1988). Pharmacopoeia of People's Republic of China (Edition 1970) records, *myo*-inositol has vitamin-like function. However, antidiabetic activity of Sequoyitol is not reported before.

### Contents of the invention

Our studies disclose that Sequoyitol is able to significantly alleviate hyperglycemia of diabetes models, inhibit the decomposition of hepatic glycogen and the absorption of glucose, reduce blood fat level, improve the metabolism of free radicals, and protect β cells of pancreatic island; does not reduce normal blood-sugar level of mice; and has extremely low toxicity. Thus, Sequoyitol can be used for prevention and treatment of diabetes and complications thereof, for prevention and treatment of metabolic disorder-associated diseases (such as hyperlipemia, fatty liver, obesity, etc.), and for improvement of the metabolism of free radicals.

The present invention further provides a process for extracting Sequoyitol from *Taxus spp.,* said process comprising: extracting *Taxus spp* with organic solvents to obtain an extract, subjecting the extract to a diphase extraction and then column chromatography to collect fractions containing Sequoyitol, then concentrating, filtrating, drying, and recrystallizing to obtain a powder containing Sequoyitol, wherein the organic solvent used for extraction comprises ethanol, methanol, acetone, and aqueous mixtures thereof, the solvents used for diphase extraction are water insoluble organic solvents, such as ethyl acetate, chloroform, dichloromethane, ethyl ether. The purification can be conducted by using various chromatographic and recrystallization methods alone or in combination manner. The solvent system of recrystallization is a solvent system comprising ethanol, acetone, methylethylketone. The chromatography may use macroporous resin columns (type D101, type MN-200, etc.), polyglucose or modified glucose columns (Sephadex G or Sephadex-LH-20, etc.), cellulose columns, activated carbon columns, etc. The final product is a crystalline powder, wherein the main effective component is Sequoyitol with a content of more than 90%.

The present invention further provides a pharmaceutical composition comprising said Sequoyitol and one or more adjuvants and/or excipients. Said pharmaceutical composition may be processed in a pharmaceutical dosage form such as injection, capsule, tablet, granule, sugar-coated pill, solution, etc.

The present invention further provides a use of said Sequoyitol in manufacture of a medicament for treatment of diabetes. Said medicament is able to significantly alleviate hyperglycemia of diabetes, inhibit the decomposition of hepatic glycogen and the absorption of glucose, reduce blood fat level, improve the metabolism of free radicals, and protect β cells of pancreatic island; and has a extremely low toxicity. Said medicament can be used for prevention and treatment of diabetes and complications in terms of diabetic cardiovessel and cerebrovessel , and glycometabolic disorder-associated diseases, for improve the metabolism of free radicals, and for prevention and treatment of type-II diabetes and complications in terms of diabetic cardiovessel and cerebrovessel .

More specifically, the process of the present invention to extract antidiabetic Sequoyitol from Taxus spp comprises: pulverizing the root, stem or leaf of Taxus spp to obtain a crude powder; extracting the crude powder with a solvent such as ethanol, methanol, acetone or aqueous mixture thereof at a temperature from 0°C to reflux temperature, preferably at a temperature from room temperature to reflux temperature, more preferably at reflux temperature, for one to five times, wherein the amount of solvent is from 1:1 to 1:20 (weight/volume), preferably from 1:2 to 1:10 (weight/volume), more preferably from 1:3 to 1:6; concentrating in vacuum to obtain an extract, subjecting said extract to a diphase extraction between water and water insoluble organic solvent (such as ethyl acetate, chloroform, dichloromethane, ethyl ether)/, and removing lipophilic organic layer, wherein the extract is preferably organic solvent/water having a ratio of from 1:0.5 to 1:10; merging water layers, filtering, and separating with chromatography, wherein the examples of chromatography column are: macroporous resin columns such as D101 type, MN-200 type (PUROLITE), etc., polyglucose G or modified polyglucose columns such as Sephadex-LH-20, etc., cellulose columns, and activated carbon columns, the corresponding eluents are used, and the elution is detected simultaneously; collecting elute fractions containing Sequoyitol, concentrating in vacuum, standing, and filtering to obtain a solid, then recrystallizing said solid with a solvent system such as ethanol, methanol, acetone, methylethylketone, and drying to obtain a Sequoyitol powder. The final product is a crystalline powder having a Sequoyitol content of more than 90%.

In the process of the present invention, the detection of fractions from the column chromatography is carried out by employing the following high performance liquid chromatography (HPLC) conditions: C18 column, 5µm, 4.6 x 250 mm; a detection wavelength of 220 nm; a sample injection of 20µl; a mobile phase of methanol-water (50:50) having a flow rate of 1.0 ml/min, which is filtrated by suction with an 0.45µm organ filtration membrane and is degassed before it is used.

The content of Sequoyitol in the product of the present invention is detected by the following HPLC method: the content was detected by precisely weighing about 25 mg the product, placing in a 25 ml volumetric flask, adding 0.65 ml dilute sulfuric acid-acetic anhydride (1:50), heating in a water bath for 20 minutes, cooling to room temperature, adding 15 ml methanol, shaking to uniformity, adding water to reach the scale, shaking to uniformity to obtain a sample solution. The HPLC conditions are the same as above.

The Sequoyitol of the present invention can be mixed with conventional adjuvants and/or excipients to form various dosage forms, such as injection, capsule, tablet, granule, sugar-coated pill, solution, etc., for prevention or treatment of diabetes, in particular type II diabetes.

The dosage forms, such as capsule, tablet, granule, sugar-coated pill, etc., can contain one or more conventional excipients , fillers and diluent : such as starch, microcrystalline cellulose, etc.; binder, such as carboxymethylcellulose, polyvinylpyrrolidone, etc.; humectant, such as glycerol; disintegrating agent, such as calcium carbonate, etc.; absorbent, kaolin, etc.; and lubricating agent, such as talc powder, etc.

The pharmaceutically acceptable excipients may be in various forms such as solid, semisolid, liquid, etc., and the adjuvants may be various types.

The solution may comprise general solvent, solubilizing agent, emulsifying agent, preservative, etc., such as water, ethanol, glycerol, polyvinyl glycol, benzyl benzoate, etc.

The dosage forms, such as capsule, tablet, granule, sugar-coated pill, solution, etc., can be prepared according to conventional methods by mixing Sequoyitol with one or more excipients.

The dosage forms, solution or emulsion, for parenteral administration should be sterile and isotonic.

### Brief description of the drawings

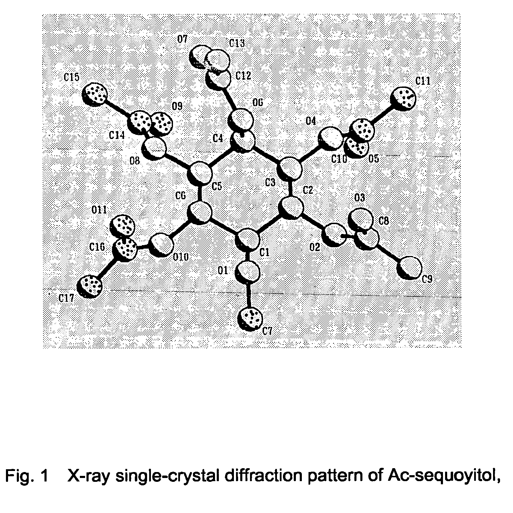

Fig. 1 is the X-ray single-crystal diffraction pattern of Ac-sequoyitol prepared according to the following process.

### Optimized Embodiments for carrying out the invention

The following examples further illustrate the present invention in more detail, but it should be understood that the examples are merely to illustrate the invention, rather than to restrict the scope of the present invention.

### Examples

### Example 1: Preparation of natural active compound Sequoyitol (1)

60 kg crude powder of Taxus spp was extracted with fourfold volume of 90% ethanol for three times, the extracts were merged and concentrated to form a viscous solution; the viscous solution was further concentrated and dried in a rotary evaporator, and diphase extracted with1:1 (v/v) chloroform/water; the aqueous layers were merged, filtrated, loaded on a macroporous resin column (D101 type, domestic-made), and gradient eluted with distilled water and aqueous ethanol (ethanol: 5% - 20%), the fractions were separately identified by HPLC, the elutes containing Sequoyitol were merged, concentrated and dried in a rotary evaporator, and then recrystallized with ethanol and filtered to obtain a crystalline powder. The crystalline powder was dried by sucking, recrystallized with 95% ethanol for twice, filtered, and dried at 70°C to obtain a Sequoyitol crystal with a yield of 0.07%.

### Example 2: Preparation of natural active compound Sequoyitol (2)

70 kg crude powder of Taxus spp was extracted with threefold volume 80% methanol for three times, the extracts were merged and concentrated to form a slurry solution; the slurry solution was further concentrated and dried in a rotary evaporator, and diphase extracted with 1:2 (v/v) ethyl acetate/water; the aqueous layers were merged, filtrated, loaded on a macroporous resin column (MN-200 type), and gradient eluted with distilled water and aqueous ethanol (ethanol: 5% - 20%), the elutes were separately identified by HPLC, the elutes containing Sequoyitol were merged, concentrated and dried by a rotary evaporator, recrystallized with acetone, and filtered to obtain a crystalline powder. The crystalline powder was dried by sucking, recrystallized with acetone : ethanol (1:1) for twice, filtered, and dried at 70°C to obtain a Sequoyitol crystal with a yield of 0.08%.

### Example 3: Preparation of natural active compound Sequoyitol (3)

65 kg crude powder of Taxus spp was extracted with fourfold volume 75% acetone for three times, the extracts were merged and concentrated to form a viscous solution; the viscous solution was further concentrated and dried in a rotary evaporator, and diphase extracted with 2:1 (v/v) dichloromethane/water; the aqueous layers were merged, filtrated, loaded on an activated carbon column (pharmaceutically acceptable standard), and gradient eluted distilled water and aqueous ethanol (ethanol: 5% - 30%), the elutes were separately identified, the elutes containing Sequoyitol were merged, concentrated and dried in a rotary evaporator, recrystallized with methanol, and filtered to obtain a crystalline powder. The crystalline powder was dried by sucking, recrystallized with methanol for twice, filtered, and dried at 70 °C to obtain a Sequoyitol crystal with a yield of 0.07%.

### Example 4: Preparation of Sequoyitol capsules

| | | |
|---|---|---|
| Prescription: | 0.2 g per capsule, comprising 25 mg Sequoyitol. | |
| | Microcrystalline cellulose | 175 g |
| | Sequoyitol | 25g |
| | | 1000 capsules |

Adjuvants were placed in a grinding container, and then Sequoyitol was added and ground for 10-30 minutes to obtain a uniform powder. The uniform powder was packaged into 1# capsules, and the content of each capsule was controlled at 0.2 g by random sampling.

### Example 5: Preparation of Sequoyitol tablets

25 g of Sequoyitol, 49 g of microcrystalline cellulose, 1 g of magnesium stearate were admixed sufficiently, and the mixture was processed by a single punch pellete to produce tablets having a diameter of 6 mm and a weight of 300 mg. Each tablet comprised 100 mg of Sequoyitol.

### Example 6: Preparation of Sequoyitol granules

20 g of Sequoyitol and 180 g of corn starch were admixed sufficiently, then an suitable amount of 60% ethanol was added to form a soft stuff. The soft stuff passed through a 12 mesh sieve, and dried to form granules. Each granule comprised 100 mg of Sequoyitol.

The chemical structure of the natural compound obtained in Example 1 was identified as follows.

### 1. Structure identification of the natural compound

The melting point of the natural compound was measured by a binocular micro-melting point detector (not corrected); the infrared spectrum was measured by a Perkin-Elemer 983 infrared spectrometer (KBr tablet); electrospray ionization mass spectrometry was measured by a LCQ type mass spectrometer of Finnigan company; optical rotation was measured by a PE-241MC type polarimeter; nuclear magnetic resonance spectrum was measured by a Brucer ACF-300 (¹H-NMR 300MHz) with TMS or DSS as internal standard.

The product obtained in Example 1 had the following physical/chemical data and spectra data: colorless crystal; MP 232-234 °C; IRν^{KBr},ₘₐₓ(cm⁻¹): 3427, 3356, 3188(OH), 1032(C-O-C); ESI(+) MS(m/z): 217.1[M+Na]⁺; ¹H-NMR (D₂O+DSS, 300MHz, ppm): 3.53(2H, dd, J=2.9, 10.0, H-1+H-3); 4.04(1 H, dd, J=2.9, 2.9, H-2); 3.67(1H, dd, J=9.8, 9.7, H-4+H-6), 3.05(1H, dd, J=9.4, 9.4, H-5); 3.59(3H, s, O-CH₃); ¹³C-NMR (D₂O+DSS, 75MHz, ppm): 86.79(CH, C-5), 74.57(CH, C-2), 74.31(CH × 2, C-4 and C-6), 73.69(CH × 2, C-1 and C-3), 62.27(OCH₃). The results indicated that the structure is 5-*O*-methyl-*myo*-inositol, i.e., Sequoyitol.

### 2. Preparation of pentaacetyl-sequoyitol (Ac-sequoyitol)

For further confirming the structure of Sequoyitol, the pentaacetylation of Sequoyitol was carried out with Ac₂O under acidic condition to prepare the pentaacetyl derivatives of Sequoyitol (Ac-sequoyitol), and its structure was identified.

Preparation of reagent: 4 ml water was added to 1.0 ml AR grade condensed sulfuric acid to obtain a sulfuric acid solution, and 0.25 ml of said sulfuric acid solution was added to 12.5 ml of Ac₂O and mixed to uniformity.

Preparation of pentaacetyl-sequoyitol (Ac-sequoyitol): 200 mg of the product of Example 1 was dissolved in 5.0 ml of said reagent, and reacted in a water bath at about 87 °C for 20 minutes; after cooled to room temperature, 100 ml of distilled water was slowly added with stirring, heated in a water both again at about 87 °C for 20 minutes; after cooled to room temperature, the reaction mixture was transferred to a separating funnel, diphase extracted with CH₂Cl₂ /H₂O for three times; the CH₂Cl₂ layers were merged, evaporated to dryness in a rotary evaporator to obtain a colorless crystalline solid; the solid was recrystallized with ethyl ether/anhydrous ethanol to obtain a colorless transparent massive crystal (i.e., Ac-sequoyitol).

### 3. Structure identification of pentaacetyl-sequoyitol (Ac-sequoyitol)

The compound Ac-sequoyitol is a colorless massive crystal; MP 201-202 °C; C₁₇H₂₄O₁₁, ESI(+)MS(m/z): 427.1[M+Na]⁺; ¹H-NMR(CDCl₃, 300MHz, ppm): 5.54 (1H, t, J=2.8, H-2); 5.45(2H, t, J=10.05, H-4 and H-6); 5.01 (2H, dd, J=2.8, 10.5, H-1 and H-3); 3.42(1 H, t, J=9.7, H-5), 3.45(3H, s, OCH₃) ; 2.16(3H, s, C₂-OAc); 2.08(6H, s, OAc×2); 1.99(6H, s, OAc×2). ³C-NMR(CDCl₃, 75MHz, ppm): 169.85 (1C, C₂-OCOCH₃), 169.63 (2C, OCOCH₃×2), 169.41 (2C, OCOCH₃×2), 80.08 (1C, C-5), 70.60 (2C, C-4+C-6), 68.78 (2C, C-1+C-3), 68.36 (1C, C-2), 60.02(1C, OCH₃), 20.39, 20.67(total 5C, OCOCH₃×5). The ¹H-¹H COSY ¹H-¹³C COSY, ¹H-¹³C COLOG long range correlated spectroscopy of Ac-sequoyitol were measured, and the ¹H and ¹³C data of said compound were assigned. Compared with Sequoyitol, the molecular weight of Ac-sequoyitol was increased by 210 (corresponding to the mass of 5 acetyl groups). The data indicated that Ac-sequoyitol was a pentaacetyl derivative of Sequoyitol and had the following structure formula:

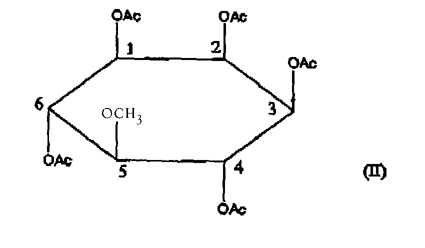

### 4. X-ray single-crystal diffraction of Ac-sequoyitol

For confirming the structure of Ac-sequoyitol, its X-ray single-crystal diffraction of Ac-sequoyitol was measured. The results of X-ray single-crystal diffraction of Ac-sequoyitol proved the structure of Ac-sequoyitol (see Fig. 1). Thus, the structure and relative configuration of Sequoyitol were confirmed. The pharmacodynamic, toxicological and general pharmacological studies of the natural compound (Sequoyitol) prepared in Example 1 were conducted as follows.

### I. Main pharmacodynamic experiments

### Experimental materials

- Animals:: Kunming mice, 20-24 g of body weight
SD rats, 230-290g of body weight, male

### Drugs and reagents:

Sequoyitol (made by the inventors)
Phenformine (positive control, Jiangsu Jintan Drug Plant)
Alloxan (Sigma Company, U.S.A.)
Glucose detection kit (Shanghai Rongsheng Biological Technology Co. Ltd.)
Glibenclamide (positive control, Tianjin Pacific Ocean Pharmaceutical Co., Ltd.)
Adrenaline Hydrochloride Injection (Wuhan Pharmaceutical Group Co., Ltd.)
Hepatic glycogen detection kit (Nanjing Jiancheng Bioengineering Institute)
50% Glucose injection (Jiangsu Changzhou Stated-owned Wujin Drug Plant) Cholesterol detection agent (Shanghai Rongsheng Biological Technology Co. Ltd.)
Malonaldehyde detection kit (Nanjing Jiancheng Bioengineering Institute)
Protein detection kit (Nanjing Jiancheng Bioengineering Institute)
Triglyceride detection kit (Shanghai Rongsheng Biological Technology Co. Ltd.)
Hepatocuprein detection kit (Nanjing Jiancheng Bioengineering Institute)
Insulin detection kit (Henan Jiaozuo Jiefang Immunodiagnosis Reagents Institute)
Streptozocin (Lot 119 H1029, Sigma Company, U.S.A.)

### I. Experiments of time-effect relation of Sequoyitol to mouse hyperglycemia induced by alloxan

### 1. Experimental methods^{[1,2]}

Alloxan was administrated to each of 30 mice by caudal vein injection with a dose of 65 mg/kg, and the mice were modeled according to the method of documents, divided into 3 groups according to blood-sugar values, wherein 2 groups were per orally administrated with 50 mg/kg of Sequoyitol and 75 mg/kg of phenformine according to 0.1 ml/10g of body weight, and the modeling group was administrated with the same volum of distilled water, separately. These administrations were conducted for continuous 7 days, and the mice were fasted for 2 hours before the last administration, and their blood-sugar levels were detected by glucose oxidase method at 1, 2, 3 and 5 hours after the last administration.

### 2. Results

As compared to the control group, the alloxan-induced hyperglycemia was alleviated after 1 hour of administration of 50 mg/kg Sequoyitol (decreased 100.0 mg/dl), the alloxan-induced hyperglycemia was significantly alleviated after 2 hours of administration (decreased 140.7 mg/dl), and the alloxan-induced hyperglycemia was highly significantly alleviated after 3 hours of administration (decreased 193.9 mg/dl) and after 5 hour of administration (decreased 174.6 mg/dl). The alloxan-induced hyperglycemia was highly significantly alleviated after 1 hour (decreased 163.1 mg/dl) and 2 hours (decreased 159.3 mg/dl) of administration of phenformine, significantly alleviated after 3 hours (decreased 104.2 mg/dl), and alleviated after 5 hours (decreased 56.6 mg/dl). Thus, the effect of alleviating hyperglycemia of 50 mg/kg Sequoyitol was higher than that of 75 mg/kg phenformine.

### II. Effects of Sequoyitol on mouse hyperglycemia induced by alloxan

### 1. Experimental methods^{[1,2]}

Among 60 mice, 10 mice were randomly selected as normal group, and the residual mice were administrated with 65 mg/kg alloxan by caudal vein injection, modeled according to the method of documents, and divided into 5 groups according to blood-sugar values, wherein 4 groups were perorally administrated with 25, 50 and 100 mg/kg of Sequoyitol and 75 mg/kg of phenformine according to 0.1 ml/10g of body weight, respectively, and the normal group and modeling group were administrated with the same volum of distilled water, separately. These administrations were conducted for continuous 7 days. The blood-sugar levels were detected by glucose oxidase method. The pancreatic glands of the mice were soaked in 10% formalin, embedded with paraffin, sliced, and dyed with HE dye.

### 2. Results

As compared to normal group, the blood-sugar level of the mice of the control group highly significantly increased (increased 308.2 mg/dl). As compared to the control group, the hyperglycemia of 25 mg/kg Sequoyitol group was alleviated, and the alloxan-induced hyperglycemia of 50 and 100 mg/kg of Sequoyitol groups and phenformine group was highly significantly alleviated (decreased 95.6 mg/dl). The effect of Sequoyitol for alleviating hyperglycemia was dose dependent. The effects of 100 mg/kg of Sequoyitol group (decreased 306.8 mg/dl) and 50 mg/kg of Sequoyitol group (decreased 195.2 mg/dl) were superior to that of 75 mg/kg phenformine group (decreased 152.8 mg/dl). The histopathologic examination of pancreatic glands indicated that the pancreatic islands of normal group were massive cords shape with clear boundary, wherein islet cells were polygonal shape with abundant cytoplasm and a central round nucleus. There were a great number of pancreatic islands and a great number of cells in islands, interstitial small vessels did not significantly change, and inflammatory cell infiltration was not obvious. As to the control group, the number of pancreatic islands decreased significantly, the size of pancreatic island reduced, the number of cells in pancreatic island decreased, and size of said cells reduced, the hyalinization of interstitial small vessels and inflammatory cell infiltration were obvious. As compared to the model group, the number of pancreatic islands and the number of islet cells of Sequoyitol groups increased significantly, while the number of pancreatic islands and the number of islet cells of phenformine group increased slightly. The results of histopathologic examination confirmed that the effect of Sequoyitol was superior to that of phenformine.

### III. Effect of Sequoyitol on blood-sugar level of normal mice

### 1. Experimental method^{[3]}

50 Mice were randomly divided into 5 groups, wherein 4 groups were orally administrated with 25, 50 and 100 mg/kg of Sequoyitol and 50 mg/kg of Glibenclamide according to 0.1 ml/10g of body weight, respectively, and the normal group was administrated with the same volum of the same volum of distilled water. These administrations were conducted for continuous 7 days. The blood-sugar levels were detected by glucose oxidase method.

### 2. Results

The blood-sugar levels of Sequoyitol groups (25, 50, 100 mg/kg) and the normal group (139.0 ± 17.8 mg/dl) were not significantly different, while the blood-sugar level of Glibenclamide group (decreased 19.3 mg/dl) was significantly lower than that of the normal group.

### IV. Effect of Sequoyitol on mouse hyperglycemia induced by adrenalin

### 1. Experimental methods^{[3]}

72 Mice were randomly divided into 6 groups, wherein 4 groups were orally administrated with 25, 50 and 100 mg/kg of Sequoyitol and 10 mg/kg of Glibenclamide according to 0.1 ml/10g of body weight, respectively, and the normal group and modeling group were administrated with the same volum of distilled water. These administrations were conducted for continuous 7 days. The normal group was administrated with the same volum of physiological saline by injection, while other groups were administrated with 0.2 mg/kg of adrenalin by intra-abdominal injection. The blood-sugar levels were detected by glucose oxidase method after 30 minutes of administration. The livers of the mice were collected, and hepatic glycogen levels were detected by anthrone method.

### 2. Results

As compared to normal group, the blood-sugar level of the mice of the control group highly significantly increased. As compared to the control group, the adrenalin-induced hyperglycemia of Sequoyitol groups (decreased 32.2, 35.5, 39.9 mg/dl) and Glibenclamide group (decreased 40.1 mg/dl) was significantly alleviated. In the meantime, the hepatic glycogen level of the control group highly significantly decreased. As compared to the control group, the 25 mg/kg of Sequoyitol group (increased 1.76 mg/g of liver tissue) and the 100 mg/kg of Sequoyitol group (increased 1.24 mg/g of liver tissue) highly increased the hepatic glycogen level, while the 50 mg/kg of Sequoyitol group (increased 1.02 mg/g of liver tissue) and Glibenclamide group (increased 1.28 mg/g of liver tissue) significantly increased the hepatic glycogen level.

### V. Effect of Sequoyitol on mouse hyperglycemia induced by glucose

### 1. Experimental method^{[4]}

72 Mice were randomly divided into 6 groups, wherein 4 groups were orally administrated with 25, 50 and 100 mg/kg of Sequoyitol and 75 mg/kg of phenformine according to 0.1 ml/10g of body weight, respectively, and the normal group and modeling group were administrated with the same volum of distilled water. These administrations were conducted for continuous 7 days. The normal group was administrated with the same volum of physiological saline by injection, while other groups were administrated with 2 mg/kg of glucose by intra-abdominal injection. After 30, 60, 90 and 120 minutes of administration, bloods were collected from venous plexus behind fossa orbitalis, the serums were separated, and the blood-sugar levels were separately detected by glucose oxidase method.

### 2. Results

As compared to normal group, the blood-sugar level of the mice of the control group highly significantly increased after 30, 60, 90, 120 minutes of administrating glucose by intra-abdominal injection. As compared to the control group, the glucose-induced hyperglycemia of 25 and 50 mg/kg Sequoyitol groups was significantly alleviated after 30, 60, 90 minutes of administrating glucose by intra-abdominal injection; the glucose-induced hyperglycemia of the 100 mg/kg of Sequoyitol group and the phenformine group was highly significantly alleviated after 30, 60, 90, 120 minutes of administrating glucose by intra-abdominal injection; the effect of Sequoyitol was essentially dose dependent, and the effect of 100 mg/kg of Sequoyitol for alleviating hyperglycemia (90 minutes, decreased 24.5 mg/dl) was equivalent to that of 75 mg/kg phenformine.

### VI. Effect of Sequoyitol on rat hyperglycemia induced by alloxan

### 1. Experimental methods^{[5,6]}

5 Rats were randomly selected as normal group, the other 55 rats were fasted for 14-16 hours, administrated with 30 mg/kg of Pentobarbital by intra-abdominal injection. After paralyzed, the rats were administrated with 48 mg/kg of alloxan by vena femoralis injection, modeled according to methods of documents, and divided into 5 groups according to blood-sugar level, wherein each group had 11 rats, and 4 groups were orally administrated with 25, 50 and 100 mg/kg of Sequoyitol and 75 mg/kg of phenformine according to 1 ml/100g of body weight, respectively, and the normal group and modeling group were administrated with the same volum of distilled water. These administrations were conducted for continuous 18 days. The fasting blood-sugar levels of the rats were separately measured at 6^{th} and 12^{th} day after the administration. At the 18^{th} day, blood was collected from arteria femoralis, and the blood sugar level, insulin level, cholesterol content, triglyceride content, malonaldehyde content, and hepatocuprein activity of serum were measured; liver tissue was homogenated, and the malonaldehyde content, and hepatocuprein activity of liver tissue were measured; and pancreatic gland was soaked in 10% formalin, embedded with paraffin, sliced, and dyed with HE.

### 2. Results

As compared to normal group, the blood-sugar level of the rats of the control group highly significantly increased. As compared to the control group, the alloxan-induced hyperglycemia of 25 mg/kg Sequoyitol group was significantly alleviated at the 12^{th} day after the administration, and highly significantly alleviated at the 18^{th} day after the administration (decreased 194.4 mg/dl). The alloxan-induced hyperglycemia of 50 mg/kg Sequoyitol group (decreased 129.1, 200.7, 223.1 mg/dl separately at 6^{th}, 12^{th} and 18^{th} day), 100 mg/kg Sequoyitol group (decreased 120, 218.2, 240.3 mg/dl separately at 6^{th}, 12^{th} and 18^{th}) and phenformine group was highly significantly alleviated since the 6^{th} day after the administration. The effect of Sequoyitol for alleviating hyperglycemia was essentially dose dependent.

As compared to normal group, the serum insulin level of rats of the control group significantly decreased. As compared to the control group, the insulin levels of 25 mg/kg Sequoyitol group (increased 1.01 µIU/ml), 50 mg/kg Sequoyitol group (increased 1.45 µIU/ml) and phenformine group increased, and the insulin levels of 100 mg/kg Sequoyitol group (increased 3.36 µIU/ml) significantly increased. The effect of Sequoyitol was essentially dose dependent.

As compared to normal group, the contents of triglyceride and cholesterol in serum of rats of the control group highly significantly increased. As compared to the control group, the contents of triglyceride and cholesterol in serum of Sequoyitol groups (50 mg/kg group, the contents of triglyceride and cholesterol in serum separately decreased 15.7 and 22.2 mg/dl) significantly or highly significantly decreased. The contents of triglyceride and cholesterol in serum of phenformine group did not significantly change.

As compared to normal group, the content of malonaldehyde in serum of rats of the control group significantly increased, and in the meantime, the content of malonaldehyde in liver tissue of rats of the control group significantly increased. As compared to the control group, the content of malonaldehyde in serum of Sequoyitol groups (100 mg/kg group, the content malonaldehyde in serum decreased 4.13 nmol/dl) highly significantly decreased. In addition, the content of malonaldehyde in liver tissue of 100 mg/kg Sequoyitol group significantly decreased (decreased 4.29 nmol/mg prot). The effect of Sequoyitol was essentially dose dependent. The contents of malonaldehyde in serum and liver tissue of phenformine group merely decreased slightly.

As compared to normal group, the activity of hepatocuprein in serum and liver tissue of rats of the control group significantly decreased. As compared to the control group, the activity of hepatocuprein in serum of Sequoyitol groups (100 mg/kg group, increased 55.0 NU/ml) increased. The activity of hepatocuprein in liver tissue of the 100 mg/kg Sequoyitol group increased (100 mg/kg group, increased 18.3 NU/mg prot). The activity of hepatocuprein in serum and liver tissue of phenformine group did not significantly change.

As compared to normal group, the body weight of rats of the control group significantly decreased. As compared to the control group, the body weight of diabetes rats of 25 mg/kg Sequoyitol group increased at the 15^{th} day, significantly increased at the 17^{th} day. The body weight of diabetes rats of 50 and 100 mg/kg Sequoyitol groups increased at the 13^{th} day, significantly increased since the 15^{th} day. The body weight of rats of phenformine group did not significantly change.

The histopathologic examination of pancreatic glands indicated that the pancreatic islands of normal group were massive himantoid shape with clear boundary, wherein islet cells were polygonal shape with abundant ketoplasm and a central round nucleus. There were a great number of pancreatic islands and a great number of cells in islands, interstitial small vessels did not significantly change, and no obvious inflammatory cell infiltration was observed. As to the control group, the number of pancreatic islands decreased significantly, the size of pancreatic island reduced, the number of cells in pancreatic island decreased, and the size of said cells reduced, the hyalinization of interstitial small vessels and inflammatory cell infiltration were obviously observed. As compared to the model group, the number of pancreatic islands and the number of cells in pancreatic islands of Sequoyitol groups increased significantly, and in 50 and 100 mg/kg Sequoyitol groups, the hyalinization of interstitial small vessels was alleviated and inflammatory cell infiltration reduced. The effect of Sequoyitol for alleviating hyalinization of interstitial small vessels was superior to that of phenformine.

### VII. Effect of Sequoyitol on mouse hyperglycemia induced by Streptozocin

### 1. Experimental methods^{[7,8]}

Among 66 mice, 10 mice were randomly selected as normal group, and the residual mice were administrated with 160 mg/kg Streptozocin by vena caudalis injection, modeled according to methods of documents, and divided into 5 groups according to blood-sugar level, wherein 4 groups were perorally administrated with 25, 50 and 100 mg/kg of Sequoyitol and 75 mg/kg of phenformine according to 0.1 ml/10g of body weight, respectively, and the normal group and modeling group were administrated with the same volum of distilled water. These administrations were conducted for continuous 18 days. The blood-sugar levels of the mice were separately measured at 6^{th} and 12^{th} day after the administration. At the 18^{th} day, blood was collected by excising eyeballs, serum was separated, and the blood sugar level, insulin level, cholesterol content, triglyceride content, malonaldehyde content, and hepatocuprein activity of serum were measured; liver tissue was homogenated, and the malonaldehyde content and hepatocuprein activity of liver tissue were measured; and pancreatic gland was soaked in 10% formalin, embedded with paraffin, sliced, and dyed with HE.

### 2. Results

As compared to normal group, the blood-sugar level of the mice of the control group highly significantly increased. As compared to the control group, the Streptozocin-induced hyperglycemia of Sequoyitol groups and phenformine group was significantly alleviated at the 12^{th} day after the administration (50 mg/kg group, decreased 121.4 mg/dl).

As compared to normal group, the serum insulin level of mice of the control group significantly decreased. As compared to the control group, the insulin levels of 25 mg/kg Sequoyitol group and phenformine group increased, and the insulin levels of 50 and 100 mg/kg Sequoyitol groups (100 mg/kg group, increased 6.53 µIU/ml) significantly increased.

As compared to normal group, the contents of triglyceride and cholesterol in serum of mice of the control group significantly increased. As compared to the control group, in 25 and 50 mg/kg Sequoyitol groups, the content of triglyceride in serum significantly decreased, and the content of cholesterol in serum decreased; in 100 mg/kg Sequoyitol group, the content of triglyceride in serum highly significantly decreased, and the content of cholesterol in serum significantly decreased. The effect of Sequoyitol was essentially dose dependent. The contents of triglyceride and cholesterol in serum of phenformine group did not significantly change.

As compared to normal group, the content of malonaldehyde in liver tissue of mice of the control group significantly increased. As compared to the control group, the content of malonaldehyde in liver tissue of 25 and 50 mg/kg Sequoyitol groups significantly decreased; and the content of malonaldehyde in liver tissue of 100 mg/kg Sequoyitol group highly significantly decreased; while the content of malonaldehyde in liver tissue of phenformine group did not significantly change.

As compared to normal group, the activity of hepatocuprein in serum of mice of the control group significantly decreased. As compared to the control group, the activity of hepatocuprein in serum of Sequoyitol groups highly significantly increased, while the activity of hepatocuprein in serum of phenformine group did not significantly change.

As compared to normal group, the body weight of mice of the control group significantly decreased. As compared to the control group, the body weight of diabetes mice of 25 mg/kg Sequoyitol group increased since the 11^{th} day. The body weight of diabetes mice of 50 and 100 mg/kg Sequoyitol groups significantly increased since the 7^{th} day, while the body weight of mice of phenformine group did not significantly change.

The histopathologic examination of pancreatic glands indicated that the pancreatic islands of normal group were massive cords shape with clear boundary, wherein islet cells were polygonal shape and has abundant cytoplasm and a central round nucleus. There were a great number of pancreatic islands and a great number of cells in islands, interstitial small vessels did not significantly change, and no obvious inflammatory cell infiltration was observed. As to the control group, the number of pancreatic islands decreased significantly, the size of pancreatic island reduced, the number of cells in pancreatic island decreased, and the size of said cells reduced, the hyalinization of interstitial small vessels and inflammatory cell infiltration were obviously observed. As compared to the model group, the number of pancreatic islands and the number of cells in pancreatic islands of Sequoyitol groups increased significantly, and the inflammatory cell infiltration was inhibited. Further, the hyalinization of interstitial small vessels in 100 mg/kg Sequoyitol group was alleviated. In the phenformine group, the number of pancreatic islands and the number of cells in pancreatic islands merely slightly increased, and the hyalinization of interstitial small vessels was not alleviated. The results of histopathologic examination confirmed that the effect of 50 and 100 mg/kg Sequoyitol was superior to that of 75 mg/kg phenformine.

### Conclusion of pharmacodynamic experiments

The pharmacodynamic studies of Sequoyitol to blood-sugar of normal mice, alloxan-induced hyperglycemia mice and rats, adrenalin model mice, glucose-induced hyperglycemia mice, and Streptozocin-induced hyperglycemia mice indicated that: Sequoyitol did not significantly affect the blood-sugar of normal mice, but significantly alleviated the hyperglycemia induced by adrenalin, increased hepatic glycogen level, had significantly sugar resistant effect to hyperglycemia induced by glucose. The blood-sugar level of body was maintained by the dynamic equilibrium between the absorption, utilization and conversion, storage of glucose in blood to control the generation and metabolism of blood sugar; besides insulin that regulates the metabolism of Carbohydrate in body to maintain blood sugar level, the decomposition and synthesis of hepatic glycogen are also important factors for regulating blood sugar level. Adrenalin does not directly destroy islet β cells, does not affect the secretion of insulin, but promotes the decomposition of hepatic glycogen and increases blood sugar level by irritating α and β receptors. Since Sequoyitol did not significantly affect the blood sugar level of normal mice, it is hinted that Sequoyitol does not significantly affect the normal glucose-metabolism, but significantly inhibits the decomposition of hepatic glycogen and the absorption of glucose.

Alloxan and Streptozocin selectively destroy islet β cells so that the secretion of insulin is insufficient, conditions like human diabetes are caused, and the animals have symptoms such as polyuria, polydipsia, extenuation, and significant blood-sugar increase. In the Alloxan and Streptozocin mice models, the levels of blood-sugar, serum triglyceride, cholesterol, malonaldehyde significantly increased, and the serum insulin level and hepatocuprein activity significantly decreased. Sequoyitol was able to decrease the elevated blood-sugar, serum triglyceride, cholesterol, and malonaldehyde contents, and to increase the serum insulin level and hepatocuprein activity (phenformine is lack of such effects). According to studies in the recent years, the metabolism of free radicals takes part in the occurrence and development of diabetes, the free radicals increase during diabetic state, the peroxidization degree of tissue lipid is seriously high, which exacerbate the tissue damage and complications. Sequoyitol improves the metabolism of free radicals, reduces the attack of free radicals at tissue and blood to lipoproteins and unsaturated fatty acid residues, and alleviates the peroxidization of lipid, so that the damage of free radicals during the occurrence and development of diabetes is alleviated. Since Sequoyitol decreased serum cholesterol and triglyceride contents, it is hinted that Sequoyitol has certain effects for prevention and treatment of complications in terms of diabetic cardiovessel and cerebrovessel , and for prevention of fatty liver in diabetics. Sequoyitol did not significantly affect the blood-sugar of normal mice, but elevated the decreased insulin level, and the results of pathological studies indicated that Sequoyitol alleviated the damage of pancreatic island caused by alloxan or Streptozocin, which hinted that Sequoyitol did not stimulate islet β cells to release insulin, but protected islet β cells and promoted the functional recovery or regeneration of islet β cells.

The above results indicate that Sequoyitol did not reduce the blood-sugar level of normal mice, but inhibited the decomposition of hepatic glycogen and the absorption of glucose, and was able to treat Alloxan and Streptozocin diabetes models, to protect islet β cells, to reduce blood fat, and to improve the metabolism of free radicals. The Sequoyitol has highly significant pharmacodynamic action, prominent features and obvious advantages, and can be used for treatment of diabetes.

References of pharmacodynamic experimental methods
1. Wu Yupeng, et al., "Hypoglycemic effects of "YiKang"", Journal of Researches on Traditional Chinese Medicine, 1997, 13(1): 57-59
2. Xu Shuyun, et al., Methodology of Pharmacology, 2nd Edition, People' Medical Publishing House, 1994: 1275-1278
3. Zhang Bing, et al., "Effects of chicory capsule on mouse blood-sugar level", Journal of Beijing University of Traditional Chinese Medicine, 1999, 22(1): 28-30
4. Chen Weihui, et al., "Effects of ophiopogonpolysaccharide on blood-sugar levels of normal and experimental diabetic mice", Chinese Journal of Pharmacology for Modern Application", 1998, 15(4): 21-23
5. Li Xiangzhong, et al., "Effects of "YiJin" hypoglycemia oral solution for reducing blood-sugar and blood-fat levels", Journal of Shengyang University of Pharmaceutical Science, 2000, 17(5): 371-374
6. Wang Shurong, et al., "Observation of effects of "Xioke Jiangtang" granules for reducing blood-sugar level of diabetic rats and for against free radicals", Pharmacology and Clinic of Chinese Medicine, 2000, 16 (supplement): 109-111
7. Yang Xiaofeng, et al., "Studies on effects of "Xiaokean" for reducing blood-sugar and blood-fat level", Chinese New Drug and Clinical Pharmacology, 1999, 10(5): 288-289
8. Zhang Juntian, Modern Pharmacological Methodology, first volume, 1st Edition, Publishing House of Beijing Medical University and China Union Medical University, 1998: 981-983

### II. Studies on toxicology

### 1. Test of acute toxicity by oral administration

### 1) Experimental method

20 Normal mice (half male and half female) were divided into two groups (each group had 10 mice), and drenched with 18.95 g/kg and 9.47 g/kg one time, wherein the higher dose reached the maximum concentration and the maximum administration volume. The animals' responses were observed. The mice were executed after 7 days, and their organs were macroscopically observed.

### 2) Experimental results

All mice did not die, and had better health status, gloss fur, bright eyes, and good rang of motion. The mice were executed after 7 days, and their main organs were free of abnormality under macroscopic observation. Since Sequoyitol has a very low toxicity or even is nontoxic, the LD₅₀ of mice by oral administration of Sequoyitol was not determined.

### 2. Test of the maximum tolerance by oral administration

### 1) Experimental method

20 Normal mice (half male and half female) were fasted and administrated with Sequoyitol twice within 24 hours, the interval period was 8 hours, and 37.9 g/kg was administrated in one day. The animals' responses were observed. The mice were executed after 14 days, and their organs were macroscopically observed.

### 2) Experimental results

All mice did not die, and had better health status, gloss fur, bright eyes, and good rang of motion. The mice were executed after 14 days, and their main organs were free of abnormality under macroscopic observation.

### 3. Test of acute toxicity by intravenous administration

### 1) Experimental method

20 Normal mice (half male and half female) were administrated with 5.04 g/kg Sequoyitol (which reached the maximum concentration and the maximum intravenous administration volume) by intravenous injection. The animals' responses were observed. The mice were executed after 14 days, and their organs were macroscopically observed.

### 2) Experimental results

All mice had better health status, gloss fur, bright eyes, and good rang of motion. The mice were executed after 14 days, and their main organs were free of abnormality under macroscopic observation. Since Sequoyitol has a very low toxicity or even is nontoxic, the LD₅₀ of mice by intravenous administration of Sequoyitol was not determined.

### References for toxicological studies

1. Guidelines for Studying New Drugs (Pharmacy, Pharmacology, Toxicology), Bureau of Drug Administration, the Ministry of Health of the People's Republic of China
2. Chen Qi, Methodology for Studying Pharmacology of Chinese Medicine, People' Medical Publishing House, 1st Edition, 1993, 118-119

### III. Studies on general pharmacology

The effects of Sequoyitol on general physical signs and spontaneous movement of normal mice, on Pentobarbital hours of sleep of mice, and on respiratory movement, blood pressure, cardiac rhythm and electrocardiogram of rats were observed. The results indicated that Sequoyitol did not significantly affect the spontaneous movement of mice, the Pentobarbital hours of sleep of mice, and the respiratory movement, blood pressure, cardiac rhythm and electrocardiogram of rats.

## Claims

1. A process for extracting a natural compound having antidiabetic effect extracted from *Taxus spp*, said process comprising: extracting *Taxus spp* with an organic solvent to obtain an extractum, subjecting the extractum to a diphase extraction and a chromatography using a macroporous resin column, collecting fractions containing *myo*-inositol derivative, then concentrating, crystallizing, and filtrating to obtain a powder, recrystallizing the powder to obtain a natural compound of 5-*O*-methyl-*myo-*inositol having the formula I: 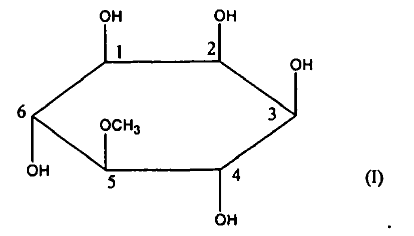 wherein the organic solvent used for extraction is selected from ethanol, methanol, acetone, and aqueous mixtures thereof; the solvent used for diphase extraction is a water insoluble organic solvent.

2. A method according to claim 1, **characterized in that** said *Taxus spp* is *Taxus yunnanensis* Cheng et L. K. Fu, or *Taxus chinensis* var. *mairei* (Lemee et Levl) Cheng et L. K. Fu.

3. A method according to claim 1, **characterized in that** the solvent used for diphase extraction is selected from ethyl acetate, chloroform, dichloromethane and ethyl ether.

4. A method according to claim 1, **characterized in that** the solvent system used for recrystallization is a solvent system selected from ethanol, methanol, acetone, methylethylketone, or a mixture thereof.

5. Use of a natural compound of 5-*O*-methyl-*myo*-inositol having the formula I in the manufacture of a medicament for treatment and/or prevention of diabetes

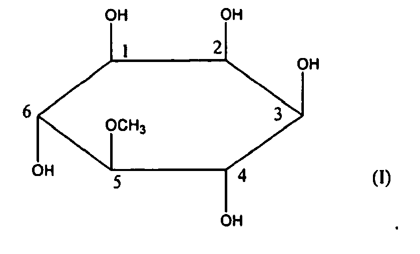

6. Use according to claim 5, **characterized in that** said medicament is able to significantly alleviate hyperglycemia of diabetes, inhibit the decomposition of hepatic glycogen and the absorption of glucose, reduce blood fat level, improve the metabolism of free radicals, and protect β cells of pancreatic island; and has a very low toxicity.

7. Use according to claim 5, **characterized in that** said medicament can be used for prevention and/or treatment of diabetes and complications in terms of diabetic cardioangiopathy and other glycometabolic disorder-associated diseases, and for improvement of the metabolism of free radicals.

8. Use according to claim 5, **characterized in that** said medicament can be used for prevention and/or treatment of type-II diabetes and complications in terms of diabetic cardioangiopathy.

9. 5-*O*-methyl-*myo*-inositol, having the formula I 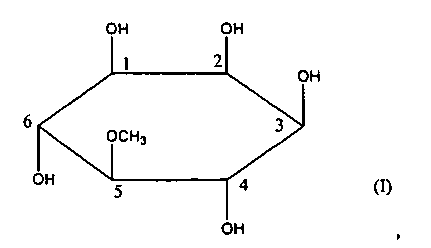 for use in a method for treatment and/or prevention of diabetes.

## Patentansprüche

1. Verfahren zur Extraktion einer natürlichen Verbindung mit antidiabetischem Effekt, die aus *Taxus spp* extrahiert wird, wobei das Verfahren umfasst: Extraktion von *Taxus spp* mit einem organischen Lösungsmittel, um ein Extrakt zu erhalten, Extraktion des Extrakts in einer Zweiphasenextraktion und Durchführen einer Chromatographie unter Verwendung einer makroporösen Harzsäule, Sammeln von Fraktionen, die ein *Myo-*Inositol-Derivat enthalten, anschließend Konzentration, Kristallisation und Filtration, um ein Pulver zu erhalten, Rekristallisation des Pulvers, um eine natürliche Verbindung von 5-*O*-Methyl-*myo*-inositol der Formel I zu erhalten: 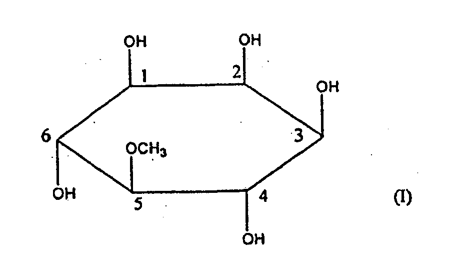 wobei das organische Lösungsmittel, das für die Extraktion verwendet wird, aus Ethanol, Methanol, Aceton und wässrigen Gemischen davon ausgewählt ist; und das Lösungsmittel, das für die Zweiphasenextraktion verwendet wird, ein wasserunlösliches organisches Lösungsmittel ist.

2. Verfahren gemäß Anspruch 1, **dadurch gekennzeichnet, dass** *Taxus spp Taxus yunnanensis* Cheng et L. K. Fu oder *Taxus chinensis* var. *mairei* (Lemee et Levl) Cheng et L. K. Fu ist.

3. Verfahren gemäß Anspruch 1, **dadurch gekennzeichnet, dass** das Lösungsmittel, das für die Zweiphasenextraktion verwendet wird, aus Ethylacetat, Chloroform, Dichlormethan und Ethylether ausgewählt ist.

4. Verfahren gemäß Anspruch 1, **dadurch gekennzeichnet, dass** das Lösungsmittelsystem, das für die Rekristallisation verwendet wird, ein Lösungsmittelsystem ist, das aus Ethanol, Methanol, Aceton, Methylethylketon oder einem Gemisch davon ausgewählt ist.

5. Verwendung einer natürlichen Verbindung von 5-*O*-Methyl-*myo*-inositol der Formel I zur Herstellung eines Medikaments zur Behandlung und/oder Vorbeugung von Diabetes

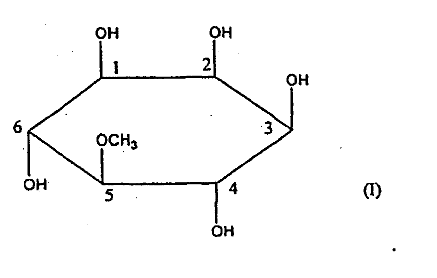

6. Verwendung gemäß Anspruch 5, **dadurch gekennzeichnet, dass** das Medikament in der Lage ist, wesentlich Hyperglykämie bei Diabetes zu verringern, den Abbau von hepatischem Glykogen und die Aufnahme von Glukose zu hemmen, den Blutfettspiegel zu senken, den Stoffwechsel freier Radikale zu verbessern und Betazellen des Inselorgans zu schützen; und eine sehr geringe Toxizität hat.

7. Verwendung gemäß Anspruch 5, **dadurch gekennzeichnet, dass** das Medikament zur Vorbeugung und/oder Behandlung von Diabetes und Komplikationen im Hinblick auf diabetische Kardioangiopathie und andere Krankheiten, die mit glykometabolischen Störungen assoziiert sind, und zur Verbesserung des Stoffwechsels freier Radikale verwendet werden kann.

8. Verwendung gemäß Anspruch 5, **dadurch gekennzeichnet, dass** das Medikament zur Vorbeugung und/oder Behandlung von Typ-II-Diabetes und Komplikationen im Hinblick auf diabetische Kardioangiopathie verwendet werden kann.

9. 5-*O*-Methyl-*myo*-inositol der Formel I 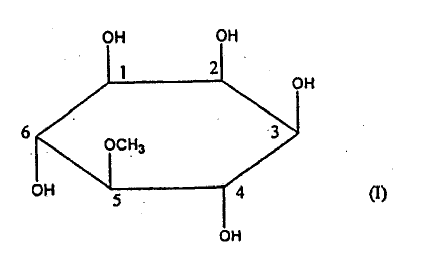 zur Verwendung in einem Verfahren zur Behandlung und/oder Vorbeugung von Diabetes.

## Revendications

1. Procédé d'extraction d'un composé naturel ayant un effet antidiabétique extrait de l'espèce *Taxus,* ledit procédé comprenant les étapes consistant à : extraire l'espèce *Taxus* avec un solvant organique pour obtenir un extrait, soumettre l'extrait à une extraction diphasique et à une chromatographie en utilisant une colonne de résine macroporeuse, à collecter les fractions contenant un dérivé *myo-inositol,* puis à concentrer, cristalliser, et filtrer pour obtenir une poudre, recristalliser la poudre pour obtenir un composé naturel de 5-*O*-méthyl-*myo*-inositol de formule I : 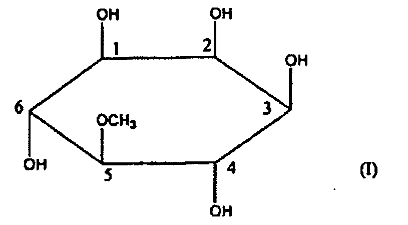 dans lequel le solvant organique utilisé pour l'extraction est choisi parmi l'éthanol, le méthanol, l'acétone, et les mélanges aqueux de ceux-ci ; le solvant utilisé pour l'extraction diphasique est un solvant organique insoluble dans l'eau.

2. Procédé selon la revendication 1, **caractérisé en ce que** ladite espèce *Taxus* est *Taxus yunnanensis* Cheng et L.K. Fu, ou *Taxus chinensis* var. *mairei* (Lemee et Levl) Cheng et L.K. Fu.

3. Procédé selon la revendication 1, **caractérisé en ce que** le solvant utilisé pour l'extraction diphasique est choisi parmi l'acétate d'éthyle, le chloroforme, le dichlorométhane et l'éthyléther.

4. Procédé selon la revendication 1, **caractérisé en ce que** le système de solvant utilisé pour la recristallisation est un système de solvant choisi parmi l'éthanol, le méthanol, l'acétone, la méthyléthylcétone ou un mélange de ceux-ci.

5. Utilisation d'un composé naturel de 5-*O*-méthyl-*myo*-inositol de formule I dans la fabrication d'un médicament pour le traitement et/ou la prévention du diabète :

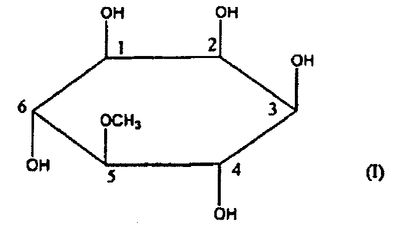

6. Utilisation selon la revendication 5, **caractérisée en ce que** ledit médicament est capable d'atténuer significativement l'hyperglycémie du diabète, d'inhiber la décomposition du glycogène hépatique et l'absorption du glucose, de réduire la teneur du sang en lipides, d'améliorer le métabolisme des radicaux libres et de protéger les cellules β de l'îlot pancréatique ; et a une très faible toxicité.

7. Utilisation selon la revendication 5, **caractérisée en ce que** ledit médicament peut être utilisé pour la prévention et/ou le traitement du diabète et de ses complications en termes de cardioangiopathie diabétique et d'autres maladies associées à des troubles glycométaboliques, et pour l'amélioration du métabolisme des radicaux libres.

8. Utilisation selon la revendication 5, **caractérisée en ce que** ledit médicament peut être utilisé pour la prévention et/ou le traitement du diabète de type II et des complications en termes de cardioangiopathie diabétique.

9. 5-*O*-méthyl-*myo*-inositol de formule I : 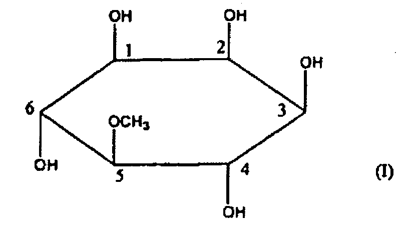 utilisable dans un procédé de traitement et/ou de prévention du diabète.
